# EUROPEAN PATENT APPLICATION

(11) **EP 4 484 940 A1**
(43) Date of publication of application: **01.01.2025**
(21) Application number: 22928884.0
(22) Date of filing: 14.11.2022
(51) Int. Cl.: G01N 27/02, C12M 1/34, C12Q 1/02

(54) **FLOW CHANNEL DEVICE**

(30) Priority: 25.02.2022 JP 2022027512
(71) Applicant: SCREEN Holdings Co., Ltd., Kyoto-shi, Kyoto 602-8585 (JP)
(72) Inventor: FUJIOKA, Ryota, Kyoto-shi, Kyoto 602-8585 (JP)
(74) Representative: Goddar, Heinz J.
(86) International application number: PCT/JP2022/042274
(87) International publication number: WO 2023/162366

(57) **Abstract**

A technique capable of easily checking the continuity of electrodes even when no liquid is put in a channel device is provided. A channel device (1) includes: a measurement vessel (2) including a measurement chamber (100) that is a channel therein; a porous membrane (30) positioned in the measurement chamber (100) and capable of supporting cellular tissue (9), the porous membrane (30) being permeable to liquid; an upper working electrode (61) and an upper reference electrode (71) both spaced upwardly apart from the porous membrane (30); and a lower working electrode (63) and a lower reference electrode (73) both spaced downwardly apart from the porous membrane (30). The upper working electrode (61) includes a first upper working portion (611) and a second upper working portion (612) which overlap the measurement chamber (100) in a vertical direction, and a first conductive pad portion (616) and second conductive pad portions (617, 618) which are exposed to the outside of the measurement vessel (2).

## Description

### Technical Field

The present invention relates to a channel device applicable to measurement of electrical resistance of cellular tissue.

### Background Art

Transepithelial electrical resistance (TEER) measurement is known as a method for evaluating the barrier function of cell layers that form a membrane structure. In the TEER measurement, an insert having a tubular shape with a bottom formed by a porous membrane is placed in a recessed portion of a culture plate, and cells are cultured on the porous membrane. Working electrodes for applying current and reference electrodes for measuring a potential difference are placed inside and outside the insert. The potential difference generated between the reference electrodes is measured while the current is applied between the working electrodes, whereby electrical resistance of the cell layers is calculated.

For example, Patent Literature 1 discloses measuring electrical resistance by inserting electrodes (10A and 10B) from one side inside and outside a culture insertion dish (21). However, for the insertion of the electrodes from one side, it is however necessary to open the top of the culture insertion dish (21). It is hence difficult to apply the technique of Patent Literature 1 to a device which does not have such an opening at the top.

On the other hand, Non Patent Literature 1 discloses a channel device in which electrodes are placed on a lid which closes a top opening of a culture vessel. Placing the electrodes on the lid in this manner allows the measurement of the electrical resistance of cell layers being cultured in a measurement chamber.

### Citation List

### Patent Literature

Patent Literature 1: Japanese Patent Application Laid-Open No. 2005-137307

### Non Patent Literature

Non Patent Literature 1: Booth R, Kim H. Characterization of a microfluidic in vitro model of the blood-brain barrier (mu BBB) Lab Chip. 2012 Apr. 24; 12(10): 1784-92.

### Summary of Invention

### Technical Problem

In the case of conventional channel devices, it is however necessary to put liquid in a channel to check the continuity of the electrodes. It has hence been structurally very difficult to detect continuity failures and the like due to breaks in the electrodes and the like when no liquid is put in the channel.

It is an object of the present invention to provide a technique capable of easily checking the continuity of electrodes even when no liquid is put in a channel device.

### Solution to Problem

To solve the aforementioned problem, a first aspect is intended for a channel device applicable to measurement of electrical resistance of cellular tissue, which comprises: a measurement vessel including a measurement chamber that is a channel therein; a porous membrane positioned in the measurement chamber and capable of supporting cellular tissue, the porous membrane being permeable to liquid; a first electrode and a second electrode both spaced toward a first side in a first direction apart from the porous membrane; and a third electrode and a fourth electrode both spaced toward a second side in the first direction apart from the porous membrane, wherein the first electrode includes a measuring electrode portion overlapping the measurement chamber in the first direction, and a first conductive pad portion and a second conductive pad portion both exposed to the outside of the measurement vessel.

A second aspect is intended for the channel device of the first aspect, wherein the measuring electrode portion extends in a second direction intersecting the first direction, wherein the second conductive pad portion is connected to the measuring electrode portion, and wherein the second conductive pad portion is spaced in the second direction apart from the first conductive pad portion.

A third aspect is intended for the channel device of the second aspect, wherein the second conductive pad portion is connected to an end portion of the measuring electrode portion as seen in the second direction.

A fourth aspect is intended for the channel device of any one of the first to third aspects, which further comprises a conductive portion positioned in the measurement vessel and extending in the first direction, wherein the first conductive pad portion is connected to the first electrode through the conductive portion.

A fifth aspect is intended for the channel device of the fourth aspect, which further comprises: a wiring portion for connecting the measuring electrode portion and the conductive portion to each other; and a third conductive pad portion connected to an intermediate portion of the wiring portion and exposed to the outside of the measurement vessel.

### Advantageous Effects of Invention

According to the channel device of the first aspect, a continuity failure of the first electrode is detected by checking the continuity between the first conductive pad portion and the second conductive pad portion.

According to the channel device of the third aspect, the continuity of the end portion of the measuring electrode portion is checked.

According to the channel device of the fourth aspect, the continuity of the conductive portion is checked.

According to the channel device of the fifth aspect, a continuity failure of the conductive portion is detected by checking the continuity between the first conductive pad portion and the third conductive pad portion.

### Brief Description of Drawings

[fig. 1] Fig. 1 is a sectional view of a channel device according to an embodiment.
[fig. 2] Fig. 2 is an exploded sectional view of the channel device according to the embodiment.
[fig. 3] Fig. 3 is a view showing upper surfaces of an upper working electrode and an upper reference electrode.
[fig. 4] Fig. 4 is a view showing upper surfaces of a lower working electrode and a lower reference electrode.
[fig. 5] Fig. 5 is a diagram showing a circuit for measuring electrical resistance of cellular tissue.

### Description of Embodiments

An embodiment according to the present invention will now be described with reference to the drawings. Components described in the embodiment are merely illustrative, and there is no intention to limit the scope of the present invention thereto. In the drawings, the dimensions and the number of components are shown in exaggeration or in simplified form, as appropriate, for the sake of easier understanding in some cases.

### <1. Embodiment>

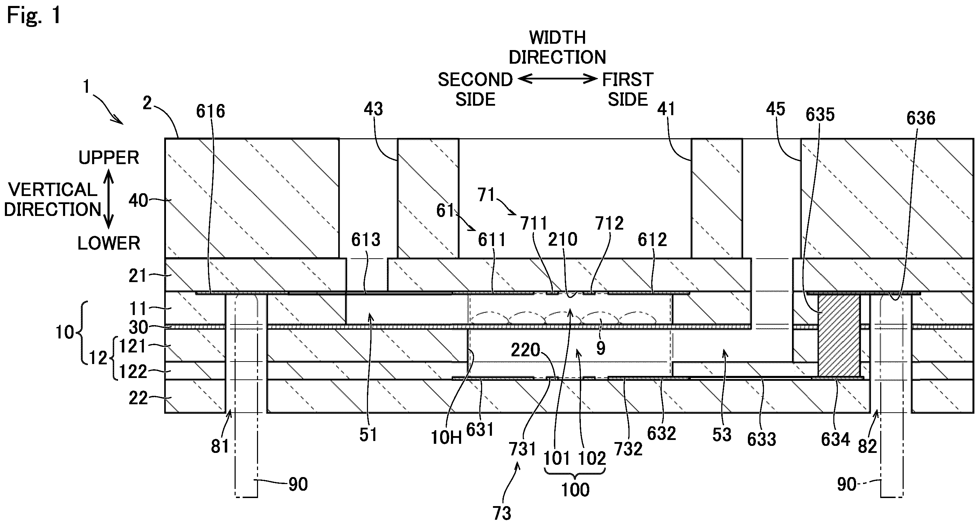

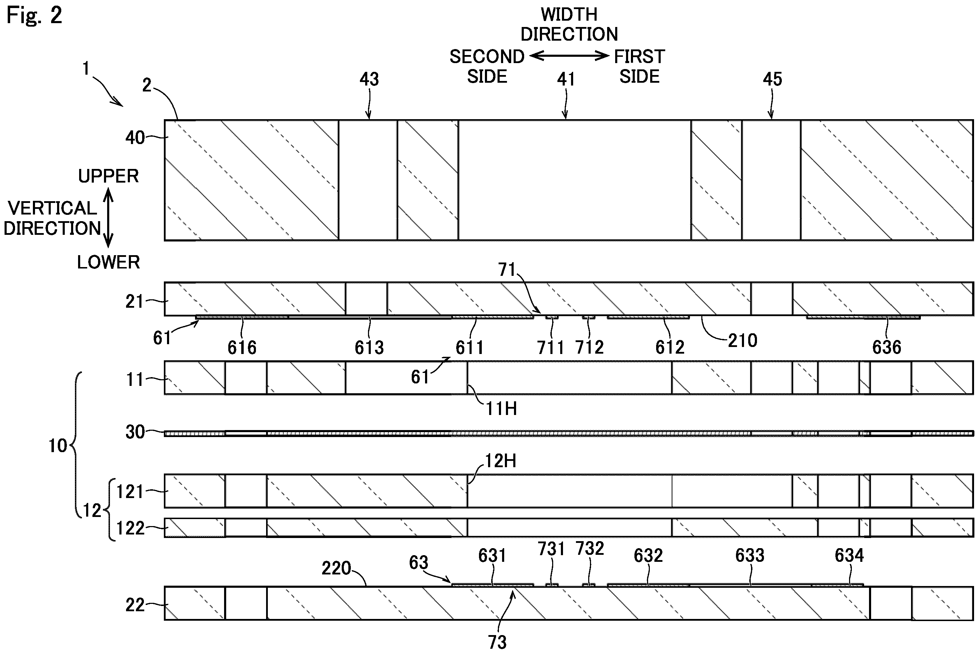

Fig. 1 is a sectional view of a channel device 1 according to an embodiment. Fig. 2 is an exploded sectional view of the channel device 1 according to the embodiment. The channel device 1 is what is called a microchannel device in which a measurement chamber 100 that is an internal space is a closed space with the exceptions of a fine first channel 51 and a fine second channel 53. The channel device 1 is applicable to a device for measuring electrical resistance (resistance, instance, or impedance) of cellular tissue 9 cultured in the measurement chamber 100 by means of a four-terminal measurement method.

As shown in Figs. 1 and 2, the channel device 1 includes a measurement vessel 2. The measurement vessel 2 includes an intermediate member 10, a first lid member 21, and a second lid member 22. The intermediate member 10, the first lid member 21, and the second lid member 22 are planar. In the channel device 1, the intermediate member 10 is located on an upper surface 220 of the second lid member 22. The first lid member 21 is located on an upper surface of the intermediate member 10.

As shown in Fig. 1, the measurement chamber 100 is located in the intermediate member 10. The measurement chamber 100 forms a channel through which liquid such as a culture solution can pass. As shown in Figs. 1 and 2, the intermediate member 10 includes a first intermediate member 11 and a second intermediate member 12. The second intermediate member 12 includes an upper member 121 and a lower member 122. The upper member 121 is located on an upper surface of the lower member 122. The first intermediate member 11 is located above the second intermediate member 12.

The intermediate member 10 is made of PET (polyethylene terephthalate), for example. The first intermediate member 11 is preferably permeable to light, and more preferably colorless and transparent. As shown in Fig. 1, the intermediate member 10 has a through hole 10H vertically passing through the intermediate member 10. An inner surface of the through hole 10H forms an inner side surface of the measurement chamber 100.

The first lid member 21 and the second lid member 22 are transparent substrates made of quartz glass, for example. As shown in Fig. 1, the first lid member 21 closes an upper opening of the measurement chamber 100. That is, an upper portion of the measurement chamber 100 is blocked by the first lid member 21. The second lid member 22 closes a lower opening of the measurement chamber 100. That is, a lower portion of the measurement chamber 100 is blocked by the second lid member 22.

As shown in Figs. 1 and 2, the channel device 1 includes a porous membrane 30. The porous membrane 30 is a sheet-like permeable membrane that is permeable to liquid. The porous membrane 30 is made of PC (polycarbonate), PTFE (polytetrafluoroethylene), or PET, for example. The porous membrane 30 preferably is permeable to light. The porous membrane 30 is located between the first intermediate member 11 and the second intermediate member 12 in a vertical direction.

The porous membrane 30 divides the measurement chamber 100 (the through hole 10H) into an upper first chamber 101 and a lower second chamber 102. As shown in Fig. 1, the cellular tissue 9 is disposed in the first chamber 101 of the measurement chamber 100 by being supported on an upper surface of the porous membrane 30 in the measurement chamber 100. An upper surface of part of the porous membrane 30 which is disposed in the measurement chamber 100 (the through hole 10H) may be coated with a cell adhesion factor (such as collagen).

As shown in Fig. 1, the first chamber 101 of the measurement chamber 100 is defined by an inner surface of a first through hole 11H which vertically passes through the first intermediate member 11, the upper surface of the porous membrane 30, and a lower surface 210 of the first lid member 21. The second chamber 102 of the measurement chamber 100 is defined by an inner surface of a second through hole 12H which vertically passes through the second intermediate member 12, a lower surface of the porous membrane 30, and the upper surface 220 of the second lid member 22.

As shown in Figs. 1 and 2, the channel device 1 includes a planar top member 40. The top member 40 is located on an upper surface of the first lid member 21. The top member 40 is made of PET, for example. The top member 40 has a viewing opening 41. The viewing opening 41 is a hole which vertically passes through the top member 40. The viewing opening 41 is located immediately over the measurement chamber 100. In other words, the viewing opening 41 overlies the measurement chamber 100 in the vertical direction.

As shown in Fig. 1, the intermediate member 10 includes the first channel 51. The first channel 51 is in communication with the first chamber 101 of the measurement chamber 100. The term "in communication" refers to a state of being coupled so that a fluid can pass through. The first channel 51 is a channel for supplying liquid such as a culture solution to the first chamber 101 of the measurement chamber 100. The first channel 51 is in the shape of a tube vertically passing through the first intermediate member 11 and surrounded by an inner surface of a through hole extending in a width direction, an upper surface of the upper member 121 of the second intermediate member 12, and the lower surface 210 of the first lid member 21. The width direction is a direction intersecting the vertical direction and is preferably orthogonal to the vertical direction.

As shown in Fig. 1, the intermediate member 10 includes the second channel 53. The second channel 53 is in communication with the second chamber 102 of the measurement chamber 100. The second channel 53 is a channel for discharging liquid from the second chamber 102 of the measurement chamber 100. The second channel 53 is in the shape of a tube vertically passing through the upper member 121 of the second intermediate member 12 and surrounded by an inner surface of a through hole extending in the width direction, a lower surface of the first intermediate member 11, and the upper surface of the lower member 122 of the second intermediate member 12.

As shown in Fig. 1, part of an upper edge of the first channel 51 is in communication with a first through hole 43 which vertically passes through the top member 40 and the first lid member 21. Also, part of an upper edge of the second channel 53 is in communication with a second through hole 45 which vertically passes through the top member 40 and the first lid member 21. Liquid can be supplied through the first through hole 43 into the measurement chamber 100. The liquid in the measurement chamber 100 can be discharged through the second through hole 45.

### <Electrodes>

As shown in Figs. 1 and 2, the channel device 1 includes an upper working electrode 61, a lower working electrode 63, an upper reference electrode 71, and a lower reference electrode 73. The upper working electrode 61 and the upper reference electrode 71 are located on the lower surface 210 of the first lid member 21. The lower working electrode 63 and the lower reference electrode 73 are located on the upper surface 220 of the second lid member 22. Each of the electrodes is formed by vacuum evaporation or the like. Part of each of the electrodes (e.g., a portion facing the inside of the measurement chamber 100) may be covered with an insulation film such as a silicon oxide film.

The upper working electrode 61, the lower working electrode 63, the upper reference electrode 71, and the lower reference electrode 73 have portions overlapping the measurement chamber 100 in the vertical direction. That is, the upper working electrode 61, the lower working electrode 63, the upper reference electrode 71, and the lower reference electrode 73 have portions overlapping the measurement chamber 100 in the vertical direction.

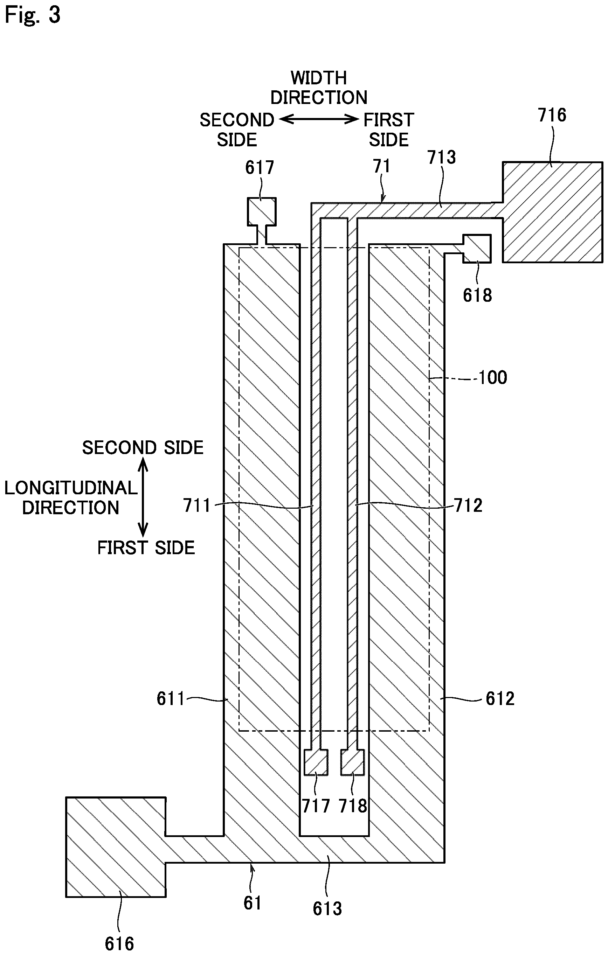

Fig. 3 is a view showing upper surfaces of the upper working electrode 61 and the upper reference electrode 71. In Fig. 3, the position of the measurement chamber 100 is shown in dash-double-dot lines.

As shown in Fig. 3, the upper working electrode 61 includes a first upper working portion 611, a second upper working portion 612, a wiring portion 613, a first conductive pad portion 616, and second conductive pad portions 617 and 618.

As shown in Fig. 3, the measurement chamber 100 extends in a longitudinal direction (a second direction). The longitudinal direction is a direction intersecting the vertical direction and the width direction. Preferably, the longitudinal direction is a direction orthogonal to the vertical direction and the width direction. The first upper working portion 611 and the second upper working portion 612 extend linearly in the longitudinal direction. The second upper working portion 612 is spaced toward a first side in the width direction apart from the first upper working portion 611.

As shown in Fig. 3, the first upper working portion 611 and the second upper working portion 612 overlap the measurement chamber 100 in the vertical direction (a first direction). The dimensions of the first upper working portion 611 and the second upper working portion 612 as measured in the longitudinal direction are greater than the dimension of the measurement chamber 100 as measured in the longitudinal direction. The first upper working portion 611 and the second upper working portion 612 extend from a position spaced toward a first side in the longitudinal direction apart from the measurement chamber 100 to a position spaced toward a second side in the longitudinal direction apart from the measurement chamber 100. In other words, the first upper working portion 611 and the second upper working portion 612 traverse the measurement chamber 100 in the longitudinal direction.

As shown in Fig. 3, the wiring portion 613 is spaced toward the first side in the longitudinal direction apart from the measurement chamber 100. The wiring portion 613 extends in the width direction. An end portion of the wiring portion 613 which is on a second side as seen in the width direction is connected to the first conductive pad portion 616. Respective end portions of the first and second upper working portions 611 and 612 which are on the first side as seen in the longitudinal direction are connected to the wiring portion 613. That is, the first upper working portion 611 and the second upper working portion 612 are electrically connected to the first conductive pad portion 616 through the wiring portion 613. The first conductive pad portion 616 is spaced toward the second side in the width direction apart from the first upper working portion 611.

The second conductive pad portions 617 and 618 are spaced toward the second side in the longitudinal direction apart from the first conductive pad portion 616. The second conductive pad portion 617 is connected to an end portion of the first upper working portion 611 which is on the second side as seen in the longitudinal direction. The second conductive pad portion 618 is connected to an end portion of the second upper working portion 612 which is on the second side as seen in the longitudinal direction. The second conductive pad portion 618 is spaced toward the first side in the width direction apart from the second upper working portion 612. The second conductive pad portions 617 and 618 are located outside the measurement chamber 100.

The upper reference electrode 71 includes a first upper reference portion 711, a second upper reference portion 712, a wiring portion 713, a first conductive pad portion 716, and second conductive pad portions 717 and 718. The first upper reference portion 711 and the second upper reference portion 712 extend linearly in the longitudinal direction. The second upper reference portion 712 is spaced toward the first side in the width direction apart from the first upper reference portion 711.

As shown in Fig. 3, the first upper reference portion 711 and the second upper reference portion 712 extend from a position spaced toward the first side in the longitudinal direction apart from the measurement chamber 100 to a position spaced toward the second side in the longitudinal direction apart from the measurement chamber 100. In other words, the first upper reference portion 711 and the second upper reference portion 712 traverse the measurement chamber 100 in the longitudinal direction.

As shown in Figs. 1 and 3, the first upper reference portion 711 and the second upper reference portion 712 are located between the first upper working portion 611 and the second upper working portion 612 as seen in the width direction. The first upper reference portion 711 is spaced toward the first side in the width direction apart from the first upper working portion 611. The second upper reference portion 712 is spaced toward the second side in the width direction apart from the second upper working portion 612.

The wiring portion 713 is spaced toward the second side in the longitudinal direction apart from the measurement chamber 100. The wiring portion 713 extends in the width direction and is connected to the first conductive pad portion 716. As shown in Fig. 3, respective end portions of the first and second upper reference portions 711 and 712 which are on the second side as seen in the longitudinal direction are connected to the wiring portion 713. That is, the first upper reference portion 711 and the second upper reference portion 712 are electrically connected to the first conductive pad portion 716 through the wiring portion 713. The first conductive pad portion 716 is spaced toward the first side in the width direction apart from the second upper reference portion 712.

The second conductive pad portions 717 and 718 are spaced toward the first side in the longitudinal direction apart from the first conductive pad portion 716. The second conductive pad portion 717 is connected to an end portion of the first upper reference portion 711 which is on the first side as seen in the longitudinal direction. The second conductive pad portion 718 is connected to an end portion of the second upper reference portion 712 which is on the first side as seen in the longitudinal direction. The second conductive pad portions 717 and 718 are located between the first upper working portion 611 and the second upper working portion 612 as seen in the width direction. The second conductive pad portions 717 and 718 are located outside the measurement chamber 100.

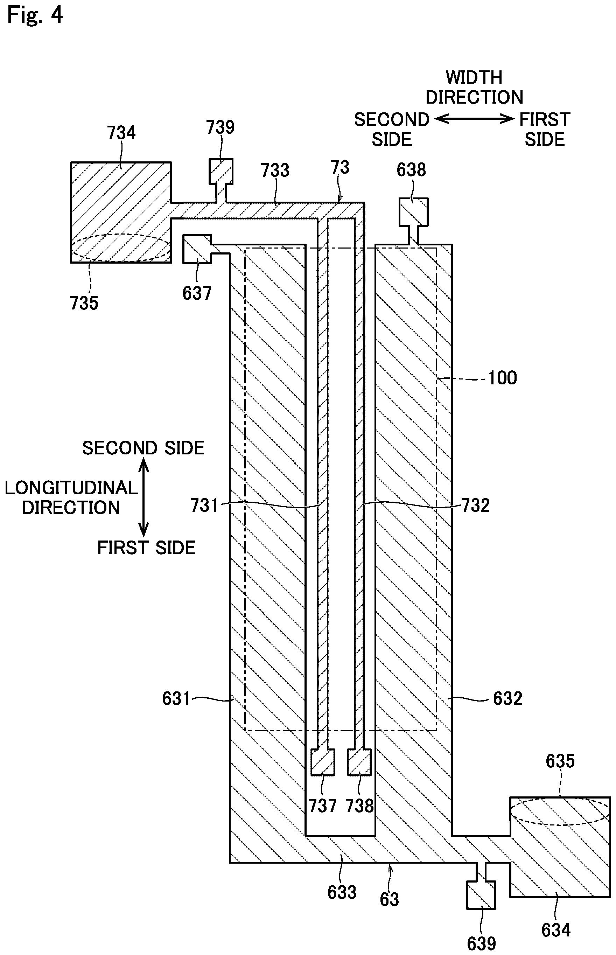

Fig. 4 is a view showing upper surfaces of the lower working electrode 63 and the lower reference electrode 73. In Fig. 4, the position of the measurement chamber 100 is shown in dash-double-dot lines.

As shown in Fig. 4, the lower working electrode 63 includes a first lower working portion 631, a second lower working portion 632, a wiring portion 633, and a relay pad portion 634.

As shown in Fig. 4, the first lower working portion 631 and the second lower working portion 632 extend linearly in the longitudinal direction. The first lower working portion 631 and the second lower working portion 632 overlap the measurement chamber 100 in the vertical direction. The first lower working portion 631 and the second lower working portion 632 traverse the measurement chamber 100 in the longitudinal direction.

The first lower working portion 631 and the second lower working portion 632 are identical in size and shape with the first upper working portion 611 and the second upper working portion 612, respectively. The first upper working portion 611 and the second upper working portion 612 are in symmetric relation to the first lower working portion 631 and the second lower working portion 632 with respect to a plane orthogonal to the vertical direction and passing through the center of the measurement chamber 100.

As seen in the vertical direction, the first lower working portion 631 is in opposed relation to the first upper working portion 611, and the second lower working portion 632 is in opposed relation to the second upper working portion 612. In this manner, the upper working electrode 61 and the lower working electrode 63 are placed in opposed relation to each other, whereby a uniform voltage is applied to the cellular tissue 9 lying between the electrodes.

As shown in Fig. 4, the wiring portion 633 is spaced toward the first side in the longitudinal direction apart from the measurement chamber 100. The wiring portion 633 extends in the width direction. An end portion of the wiring portion 633 which is on the first side as seen in the width direction is electrically connected to the relay pad portion 634. Respective end portions of the first and second lower working portions 631 and 632 which are on the first side as seen in the longitudinal direction are connected to the wiring portion 633. That is, the first lower working portion 631 and the second lower working portion 632 are electrically connected to the relay pad portion 634 through the wiring portion 633. The relay pad portion 634 is spaced toward the first side in the width direction apart from the second lower working portion 632.

As shown in Fig. 1, the lower working electrode 63 further includes a conductive portion 635 and a first conductive pad portion 636. The conductive portion 635 is a conductive member extending in the vertical direction. The conductive portion 635 is, for example, a silver paste. The conductive portion 635 is located in a hole which passes through the first intermediate member 11, the second intermediate member 12, and the porous membrane 30 in the vertical direction. The first conductive pad portion 636 is a conductive film provided on the lower surface 210 of the first lid member 21. The first conductive pad portion 636 overlaps the relay pad portion 634 in the vertical direction. As shown in Fig. 1, a lower end portion of the conductive portion 635 contacts the relay pad portion 634, and an upper end portion of the conductive portion 635 contacts the first conductive pad portion 636. The relay pad portion 634 and the first conductive pad portion 636 are electrically connected to each other through the conductive portion 635.

As shown in Fig. 4, the lower working electrode 63 includes second conductive pad portions 637 and 638. The second conductive pad portions 637 and 638 are located on the upper surface 220 of the second lid member 22. The second conductive pad portions 637 and 638 are spaced toward the second side in the longitudinal direction apart from the relay pad portion 634. The second conductive pad portions 637 and 638 are also spaced toward the second side in the longitudinal direction apart from the first conductive pad portion 636.

The second conductive pad portion 637 is connected to an end portion of the first lower working portion 631 which is on the second side as seen in the longitudinal direction. The second conductive pad portion 638 is connected to an end portion of the second lower working portion 632 which is on the second side as seen in the longitudinal direction. The second conductive pad portion 637 is located on the second side as seen in the width direction relative to the first lower working portion 631. The second conductive pad portions 637 and 638 are located outside the measurement chamber 100.

As shown in Fig. 4, the lower working electrode 63 includes a third conductive pad portion 639. The third conductive pad portion 639 is located on the upper surface 220 of the second lid member 22. The third conductive pad portion 639 is connected to an intermediate portion of the wiring portion 633. Specifically, the third conductive pad portion 639 is connected at a position between a portion of the wiring portion 633 to which the second lower working portion 632 is connected and a portion of the wiring portion 633 to which the relay pad portion 634 is connected.

As shown in Fig. 4, the lower reference electrode 73 includes a first lower reference portion 731, a second lower reference portion 732, a wiring portion 733, and a relay pad portion 734.

As shown in Fig. 4, the first lower reference portion 731 and the second lower reference portion 732 extend linearly in the longitudinal direction. The first lower reference portion 731 and the second lower reference portion 732 overlap the measurement chamber 100 in the vertical direction. The first lower reference portion 731 and the second lower reference portion 732 traverse the measurement chamber 100 in the longitudinal direction.

The first lower reference portion 731 and the second lower reference portion 732 are identical in size and shape with the first upper reference portion 711 and the second upper reference portion 712, respectively. The first upper reference portion 711 and the second upper reference portion 712 are in symmetric relation to the first lower reference portion 731 and the second lower reference portion 732 with respect to a plane orthogonal to the vertical direction and passing through the center of the measurement chamber {measurement chamber 100}. As seen in the vertical direction, the first lower reference portion 731 is in opposed relation to the first upper reference portion 711, and the second lower reference portion 732 is in opposed relation to the second upper reference portion 712.

As shown in Fig. 4, the wiring portion 733 is spaced toward the second side in the longitudinal direction apart from the measurement chamber 100. The wiring portion 733 extends in the width direction. An end portion of the wiring portion 733 which is on the second side as seen in the width direction is connected to the relay pad portion 734. Respective end portions of the first and second lower reference portions 731 and 732 which are on the second side as seen in the longitudinal direction are connected to the wiring portion 733. That is, the first lower reference portion 731 and the second lower reference portion 732 are electrically connected to the relay pad portion 734 through the wiring portion 733. The relay pad portion 734 is spaced toward the second side in the width direction apart from the first lower reference portion 731.

The lower reference electrode 73 includes a conductive portion 735 (with reference to Fig. 4). The conductive portion 735 is a conductive member extending in the vertical direction. The conductive portion 735 is, for example, a silver paste. Like the conductive portion 635, the conductive portion 735 is located in a hole (not shown) which passes through the first intermediate member 11, the second intermediate member {second intermediate member 12}, and the porous membrane 30 in the vertical direction. A lower end of the conductive portion 735 contacts the relay pad portion 734. An upper end of the conductive portion 735 contacts a first conductive pad portion (not shown) located on the lower surface 210 of the first lid member 21. The first conductive pad portion of the lower reference electrode 73 is exposed to the outside of the measurement vessel 2 through a through hole (not shown) provided in the measurement vessel 2.

As shown in Fig. 4, the lower reference electrode 73 includes second conductive pad portions 737 and 738. The second conductive pad portions 737 and 738 are located on the upper surface 220 of the second lid member 22. The second conductive pad portions 737 and 738 are spaced toward the first side in the longitudinal direction apart from the relay pad portion 734. The second conductive pad portions 737 and 738 are also spaced toward the first side in the longitudinal direction apart from the first conductive pad portion of the lower reference electrode 73.

The second conductive pad portion 737 is connected to an end portion of the first lower reference portion 731 which is on the first side as seen in the longitudinal direction. The second conductive pad portion 738 is connected to an end portion of the second lower reference portion 732 which is on the first side as seen in the longitudinal direction. The second conductive pad portions 737 and 738 are located between the first lower working portion 631 and the second lower working portion 632 as seen in the width direction. The second conductive pad portions 737 and 738 are located outside the measurement chamber 100.

As shown in Fig. 4, the lower reference electrode 73 includes a third conductive pad portion 739. The third conductive pad portion 739 are located on the upper surface 220 of the second lid member 22. The third conductive pad portion 739 is connected to an intermediate portion of the wiring portion 733. Specifically, the third conductive pad portion 739 is connected at a position between a portion of the wiring portion 733 to which the first lower reference portion 731 is connected and a portion of the wiring portion 733 to which the relay pad portion 734 is connected.

As shown in Fig. 1, the measurement vessel 2 includes conduction holes 81 and 82. Each of the conduction holes 81 and 82 is defined by an inner surface of a hole which passes through the intermediate member 10, the porous membrane 30, and the second lid member 22 in the vertical direction, and the lower surface 210 of the first lid member 21 which closes an upper portion of the through hole {hole}. The conduction hole 81 is spaced toward the second side in the width direction apart from the measurement chamber 100. The conduction hole 82 is spaced toward the first side in the width direction apart from the measurement chamber 100. As shown in Fig. 1, each of the conduction holes 81 and 82 allows a conductive probe pin 90 (external electrode) to be inserted therein.

As shown in Fig. 1, the first conductive pad portion 616 of the upper working electrode 61 is located in the conduction hole 81. Also, the first conductive pad portion 636 of the lower working electrode 63 is located in the conduction hole 82. That is, the first conductive pad portions 616 and 636 are exposed to the outside of the measurement vessel 2 through the conduction holes 81 and 82, respectively.

The measurement vessel 2 includes conduction holes (not shown) which expose the second conductive pad portions 617 and 618, respectively, of the upper working electrode 61 to the outside of the measurement vessel 2. The measurement vessel 2 further includes conduction holes (not shown) which expose the second conductive pad portions 637 and 638 and the third conductive pad portion 639, respectively, of the lower working electrode 63 to the outside of the measurement vessel 2.

The measurement vessel 2 includes conduction holes (not shown) which expose the first conductive pad portion 716 of the upper reference electrode 71 and the second conductive pad portions 737 and 738, respectively, to the outside of the measurement vessel 2. The measurement vessel 2 further includes conduction holes (not shown) which expose a first conductive pad portion 736, the second conductive pad portions 737 and 738, and the third conductive pad portion 739, respectively, of the lower reference electrode 73 to the outside of the measurement vessel 2.

As shown in Fig. 1, the probe pin 90 is inserted in each of the conduction holes including the conduction holes 81 and 82 in the measurement vessel 2. This electrically connects the conductive pads of the upper working electrode 61, the lower working electrode 63, the upper reference electrode 71, and the lower reference electrode 73 to external devices.

In the present embodiment, the upper working electrode 61 is an example of a "first electrode"; the upper reference electrode 71 is an example of a "second electrode"; the lower working electrode 63 is an example of a "third electrode"; and the lower reference electrode 73 is an example of a "fourth electrode". The first upper working portion 611 and the second upper working portion 612 are examples of a "measuring electrode portion".

The upper reference electrode 71 may be taken as the "first electrode" and the first upper reference portion 711 and the second upper reference portion 712 may be taken as the "measuring electrode portions". In this case, the upper working electrode 61 corresponds to the "second electrode", and the lower working electrode 63 and the lower reference electrode 73 correspond to the "third electrode" and the "fourth electrode", respectively.

The lower working electrode 63 may be taken as the "first electrode" and the first lower working portion 631 and the second lower working portion 632 may be taken as the "measuring electrode portions". In this case, the lower reference electrode 73 corresponds to the "second electrode", and the upper working electrode 61 and the upper reference electrode 71 correspond to the "third electrode" and the "fourth electrode", respectively.

The lower reference electrode 73 may be taken as the "first electrode" and the first lower reference portion 731 and the second lower reference portion 732 may be taken as the "measuring electrode portions". In this case, the lower working electrode 63 corresponds to the "second electrode", and the upper working electrode 61 and the upper reference electrode 71 correspond to the "third electrode" and the "fourth electrode", respectively.

### <Continuity Check>

The channel device 1 is capable of checking the continuity of the upper working electrode 61, the lower working electrode 63, the upper reference electrode 71, and the lower reference electrode 73 even in the absence of liquid such as a culture solution in the measurement chamber 100 serving as the channel.

In the upper working electrode 61, for example, a continuity failure of the first upper working portion 611 and the wiring portion 613 is detectable by checking the continuity between the first conductive pad portion 616 and the second conductive pad portion 617 shown in Fig. 3. Also, a continuity failure of the second upper working portion 612 and the wiring portion 613 is detectable by checking the continuity between the first conductive pad portion 616 and the second conductive pad portion 618.

In the upper reference electrode 71, a continuity failure of the first upper reference portion 711 and the wiring portion 713 is detectable by checking the continuity between the first conductive pad portion 716 and the second conductive pad portion 717 shown in Fig. 3. Also, a continuity failure of the second upper reference portion 712 and the wiring portion 713 is detectable by checking the continuity between the first conductive pad portion 716 and the second conductive pad portion 718.

In the lower working electrode 63, a continuity failure of the first lower working portion 631 and the wiring portion 633 is detectable by checking the continuity between the second conductive pad portion 637 and the third conductive pad portion 639 shown in Fig. 4. Also, a continuity failure of the second lower working portion 632 and the wiring portion 633 is detectable by checking the continuity between the second conductive pad portion 638 and the third conductive pad portion 639.

In the lower working electrode 63, a continuity failure (a contact failure, a break, or the like) of the relay pad portion 634, the conductive portion 635, and the first conductive pad portion 636 is detectable by checking the continuity between the first conductive pad portion 636 and the third conductive pad portion 639.

A continuity failure between the pad portions is more or less detectable by checking the continuity between the second conductive pad portion 637 or the second conductive pad portion 638 and the first conductive pad portion 636. However, it is difficult to distinguish between the continuity failure of the first lower working portion 631 or the second lower working portion 632 and the continuity failure of the conductive portion 635. On the other hand, the provision of the third conductive pad portion 639 allows separate checks of the continuity failure of the first lower working portion 631 or the second lower working portion 632 and the continuity failure of the conductive portion 635. Thus, the location of the continuity failure is easily identified.

In the lower reference electrode 73, a continuity failure of the first lower reference portion 731 and the wiring portion 733 is detectable by checking the continuity between the second conductive pad portion 737 and the third conductive pad portion 739 shown in Fig. 4. Also, a continuity failure of the second lower reference portion 732 and the wiring portion 733 is detectable by checking the continuity between the second conductive pad portion 738 and the third conductive pad portion 739.

In the lower reference electrode 73, a continuity failure (a contact failure, a break, or the like) of the relay pad portion 734, the conductive portion 735, and the first conductive pad portion (not shown) is detectable by checking the continuity between the first conductive pad portion and the third conductive pad portion 739.

### <Electrical Resistance Measurement>

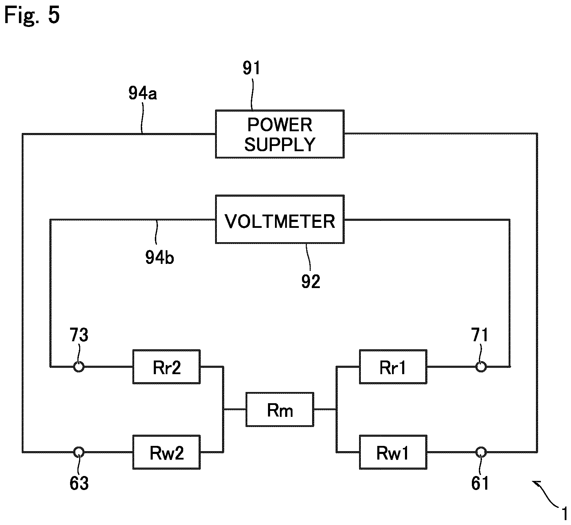

Fig. 5 is a diagram showing a circuit for measuring the electrical resistance of the cellular tissue 9. For the measurement of the electrical resistance of the cellular tissue 9, a power supply device 91 and a voltmeter 92 are connected to the channel device 1. Specifically, the power supply device 91 has an output terminal electrically connected through a conductor 94a to the upper working electrode 61 and the lower working electrode 63. The voltmeter 92 has an input terminal electrically connected through a conductor 94b to the upper reference electrode 71 and the lower reference electrode 73. The conductors 94a and 94b are connected to the electrodes through the probe pins 90.

The cellular tissue 9 is held on the upper surface of the porous membrane 30 in the measurement chamber 100. A supplying tube is connected to the first through hole 43 of the top member 40 to supply liquid (such as a culture solution) into the measurement chamber 100. The liquid is supplied through the supplying tube to the first channel 51 and the measurement chamber 100. A discharging tube is connected to the second through hole 45 of the top member 40. The liquid is discharged from the measurement chamber 100 and the second channel 53 through the discharging tube. As a result, the liquid is exchanged (or circulated) in the first and second chambers 101 and 102 of the measurement chamber 100, as appropriate.

With reference to Fig. 5, a resistance Rm corresponds to the electrical resistance of a portion of the porous membrane 30 which is disposed in the measurement chamber 100 and the cellular tissue 9 supported by the porous membrane 30 (which are referred to hereinafter as "cellular portions"). A resistance Rw1 corresponds to the electrical resistance of the liquid between the upper working electrode 61 and the cellular portions (specifically, the liquid in the first chamber 101 of the measurement chamber 100). A resistance Rw2 corresponds to the electrical resistance of the liquid between the lower working electrode 63 and the cellular portions (specifically, the liquid in the second chamber 102).

With reference to Fig. 5, a resistance Rr1 corresponds to the electrical resistance of the liquid between the upper reference electrode 71 and the cellular portions (specifically, the liquid in the first chamber 101 of the measurement chamber 100). A resistance Rr2 corresponds to the electrical resistance of the liquid between the lower reference electrode 73 and the cellular portions (specifically, the liquid in the second chamber 102 of the measurement chamber 100).

The power supply device 91 applies voltage between the upper working electrode 61 and the lower working electrode 63. The voltmeter 92 measures voltage between the upper reference electrode 71 and the lower reference electrode 73. The precise voltage value between the upper working electrode 61 and the lower working electrode 63 is calculated from the measured voltage value by a computer not shown. The resistance Rm of the cellular portions is calculated from the calculated voltage value. The electrical resistance of the cellular tissue 9 is determined by subtracting the resistance value of the porous membrane 30 from the resistance Rm of the cellular portions. The resistance value of the porous membrane 30 is determined by measuring the electrical resistance in the absence of the cellular tissue 9.

When voltage is applied between the upper working electrode 61 and the lower working electrode 63, the oxidation and reduction reactions of the liquid occur on surfaces of the upper and lower working electrodes 61 and 63 to form an electric double layer in some cases. When the electric double layer is formed, there is apprehension that the voltage applied between the upper working electrode 61 and the lower working electrode 63 differs from an output voltage caused by the power supply device 91. On the other hand, the upper and lower reference electrodes 71 and 73 are disposed in the vicinity of the upper and lower working electrodes 61 and 63 in the measurement chamber 100. Thus, the voltage between the upper working electrode 61 and the lower working electrode 63 is approximately acquired from the measured voltage value between the upper reference electrode 71 and the lower reference electrode 73. This allows the accurate measurement of the resistance Rm of the cellular portions.

### <2. Modifications>

While the embodiment according to the present invention has been described hereinabove, the present invention is not limited to the aforementioned embodiment, but various modifications may be made.

For example, the conductive portion 635 of the lower working electrode 63 may be omitted. In this case, for example, a through hole which exposes the relay pad portion 634 to the outside of the measurement vessel 2 may be provided in the measurement vessel 2 to thereby cause the relay pad portion 634 to function as the first conductive pad portion 636.

While the invention has been shown and described in detail, the foregoing description is in all aspects illustrative and not restrictive. It is therefore understood that numerous modifications and variations can be devised without departing from the scope of the invention. The components described in the aforementioned embodiment and in the various modifications may be combined together or dispensed with, as appropriate, unless the components are inconsistent with each other.

### Reference Signs List

1 Channel device
100 Measurement chamber
2 Measurement vessel
30 Porous membrane
61 Upper working electrode
611 First upper working portion
612 Second upper working portion
613 Wiring portion
616 First conductive pad portion
617, 618 Second conductive pad portions
63 Lower working electrode
631 First lower working portion
632 Second lower working portion
633 Wiring portion
634 Relay pad portion
635 Conductive portion
636 First conductive pad portion
637, 638 Second conductive pad portions
639 Third conductive pad portion
71 Upper reference electrode
711 First upper reference portion
712 Second upper reference portion
713 Wiring portion
716 First conductive pad portion
717, 718 Second conductive pad portions
73 Lower reference electrode
731 First lower reference portion
732 Second lower reference portion
733 Wiring portion
735 Conductive portion
736 First conductive pad portion
737, 738 Second conductive pad portions
739 Third conductive pad portion
9 Cellular tissue

## Claims

1. A channel device applicable to measurement of electrical resistance of cellular tissue, comprising:
a measurement vessel including a measurement chamber that is a channel therein;
a porous membrane positioned in said measurement chamber and capable of supporting cellular tissue, said porous membrane being permeable to liquid;
a first electrode and a second electrode both spaced toward a first side in a first direction apart from said porous membrane; and
a third electrode and a fourth electrode both spaced toward a second side in said first direction apart from said porous membrane,
wherein said first electrode includes
a measuring electrode portion overlapping said measurement chamber in said first direction, and
a first conductive pad portion and a second conductive pad portion both exposed to the outside of said measurement vessel.

2. The channel device according to Claim 1,
wherein said measuring electrode portion extends in a second direction intersecting said first direction,
wherein said second conductive pad portion is connected to said measuring electrode portion, and
wherein said second conductive pad portion is spaced in said second direction apart from said first conductive pad portion.

3. The channel device according to Claim 2,
wherein said second conductive pad portion is connected to an end portion of said measuring electrode portion as seen in said second direction.

4. The channel device according to any one of Claims 1 to 3, further comprising
a conductive portion positioned in said measurement vessel and extending in said first direction,
wherein said first conductive pad portion is connected to said first electrode through said conductive portion.

5. The channel device according to Claim 4, further comprising:
a wiring portion for connecting said measuring electrode portion and said conductive portion to each other; and
a third conductive pad portion connected to an intermediate portion of said wiring portion and exposed to the outside of said measurement vessel.
